# EUROPEAN PATENT APPLICATION

(11) **EP 3 560 522 A1**
(43) Date of publication of application: **30.10.2019**
(21) Application number: 18382292.3
(22) Date of filing: 27.04.2018
(51) Int. Cl.: A61L 9/014, B01D 39/00, B01D 53/02

(54) **AIR PURIFYING DEVICE USING FILTERING MICROPARTICLES AND AN ADHESIVE**

(71) Applicant: Taurus Research and Development S.L.U., 25790 Oliana (ES)
(72) Inventor: TRENCH ROCA, Lluís, 08650 SALLENT (ES); BOSCH HEREU, Lluís, 08460 Santa Maria de Palautordera (ES); VILARRASA LLORENS, Jaume, 08029 Barcelona (ES)
(74) Representative: Juncosa Miró, Jaime

(57) **Abstract**

An air purifying device (1) through which a controlled flow of air is conveyed by an air impeller device (2) at a maximum limiting given speed, through a housing (3) with an air inlet (31) and an air outlet (32). The air purifying device (1) includes a main filter (4) which acts as an adsorption and an absorption filter, and is made up of a mixture of microparticles of activated carbon (41), a plurality of filament-like elements (42), either inorganic or organic fibres, coated with an adhesive agent (45), which allows for a higher controlled speed of the airflow, and a reducing metal (43), in a sponge-like mesh configuration (5).

## Description

### Technical field

The present invention relates to an air purifying device for reducing, eliminating or cleansing contaminating agents from an air flow from an ambient in a human environment, which is suitable for domestic use such as households, apartment buildings and other residential areas. The cited air purifying device can also be useful for hospitals, schools, hotels, department stores, leisure or entertainment areas (such as theatres, cinemas or pubs), and asthmatic, clean or hypoallergenic rooms.

Other intended applications may involve industrial air filtering applications or devices installed in open areas, such as streets, roads or parks which are near known focus points emitting contaminating agents.

Furthermore, the present invention relates specifically to a main filter of the air purifying device and the particular composition of this main filter, which allows it to cleanse a flow of air by filtering and eliminating certain contaminating agents (primarily gases and vapours) that are harmful to humans in certain concentrations, by adsorption and mainly by absorption.

In addition, throughout this document, the definition of the term "particle size" should be considered as a rough approximation of the diameter of a spheroid-like shaped particle, wherein the units used are microns (also known as micrometres), which may also be represented as "µm", and wherein 1 micron (µm) is equivalent to 1000 nm (nanometres) or 0.001 mm (millimetres).

The term activated carbon (AC) is equivalent to the following known terms also used in the field: active carbon and activated or active charcoal. The use of the acronym AC may be used throughout this document.

The term "filament-like element" should be understood herein as a slender thread-like object or fibre, also including narrow or thin strips of an elongated material, such as tape, wherein these filament-like elements are capable of entangling themselves forming a sponge-like mesh configuration which acts as a support or carrier for the particles. The word element used in the term "filament-like element" should not be confused or understood as chemical element.

### State of the Art

In recent decades, the need for effective air purifying devices has risen due to the exponential increase regarding the presence of contaminating or polluting agents in the environment, such as:
- airborne particulate matter (PM_{2.5}, PM₁₀) including dust, pollen, heavy metals or bacteria;
- gases such as ozone (O₃), nitrogen oxides (NO_{X}) or sulphur oxides (SO_{X}); and
- Volatile Organic Compounds (VOCs).

US patent number 5,462,693 (from now on US'693) describes an air purifying agent comprising a carrier with one or more chemical components, in order to purify environmentally polluted air in industries, working environments or living environments.

The main component of the air purifying agent described in US'693 comprises a carrier containing short wood pulp fibre with one or more chemical components, selected from: an oxidizing agent, an acid, an alkali, or an antimicrobial component. Furthermore, the air purifying agent contains: synthetic short fibres selected from the group comprising glass fibre, silica fibre, slag wool, polyester, polyamide, polypropylene, polyacrylonitriles and rayon, and an insoluble inorganic powder selected from a group comprising activated carbon (AC) and phosphates of calcium, magnesium, aluminium, iron, zinc, titanium, zirconium, manganese and copper. When activated carbon is used as the support or carrier, it is used in pellet form with a particle size between 3 and 5 mm.

US patent number 6,641,648 (from now on US'648) divulges a filter system for passive filtration of chemical or biological warfare agents and other toxic agents, as well as the usual indoor air contaminants such as VOCs, aerosols and airborne particles (such as dust or allergens), which may be present in commercial or military facilities. It also presents water adsorption inhibiting characteristics.

The passive filter system disclosed in US'648 comprises: a housing with an air intake and an air outlet; a pleated carbon active filter which may be dispersed with at least one of a first hydrophobic and/or anti-microbial solution; a second solution that includes one or more reactive metals or a gas absorbing solution; a high efficiency particle filter adjacent to the intake of the housing for aerosols; a blower at the outlet to increase the flow of air; and optionally one or more lower efficiency filters adjacent to said high efficiency filter.

The pleated carbon active filter is made up of AC fibres with a diameter between 20 and 30 microns which are impregnated with the solutions mentioned previously.

Other well-known types of filters, used nowadays, include filters made up of a plurality of folded layers or a honeycomb structured layer of fibres, usually glass or mineral wool fibres, to which AC in granular or pelletized form is added. However, when AC is in a granular or pelletized state, the functional surface area is severely diminished, as only the surface of these granules or pellets of AC interact with the contaminants or polluting agents in the airflow, and a large quantity of AC, which is inside the granule or pellet, mixed with a binder or thickener agent, is wasted.

Furthermore, these granules or pellets of AC are unable to cover the whole extension of the layer of fibres to which they are adhered, in some cases through the use of adhesives or binding agents, due to their size, and therefore regardless of the structure or configuration used, there are areas of the filter through which airborne contaminating agents, primarily gases or vapours and airborne particles, flow through freely without interacting with the granules or pellets of AC, and therefore go back into the environment unaltered.

The present invention aims to solve the issues encountered in the present state of the art regarding the effective use of the maximum surface area of AC particles, which will therefore lead to a substantial decrease of contaminants that flow back into the environment.

In fact, it is known that 1 gram of AC powder may have a surface area of up to 3000 m², when applied for gas adsorption. Therefore, there is an indirect proportional relation between the size of particles of AC used and the effectiveness of the surface area, which is one of the main issues to be addressed with the present invention, wherein the use of small-sized particles of AC will lead to a higher capture efficiency per gram of AC, using the maximum surface area available of the AC particles.

### Brief description of the invention

In order to solve the issues raised previously, the present invention describes an air purifying device, through which a controlled flow of air (limited to a maximum speed) is filtered, from a contaminated environment or atmosphere, present in domestic environments, but it may also be suitable for offices and other work premises, hospitals, schools, hotels, rooms which require certain specific conditions such as asthmatic or hypoallergic rooms or entertainments or leisure areas (department stores, cinemas, theatres, pubs, etc.). The contaminated environment or atmosphere mainly includes:
- airborne particulate matter (PM_{2.5}, PM₁₀);
- gases such as ozone (O₃), nitrogen oxides (NO_{X}, specifically NO₂ and N₂O_{X} wherein x is a value between 3 and 5) or sulphur oxides (SO_{X}, specifically SO₂ and SO₃); and
- certain Volatile Organic Compounds (VOCs), which are the cause of many bad odours and in some cases, they are toxic.

The cited air purifying device includes an air impeller device providing a controlled flow of air, comprising at least a motor, or other equivalent actuator means, and an element capable of creating a controlled airflow, such as a centrifugal fan, an air propeller, an air aspirator or other means which can provide the controlled airflow, and having a housing which comprises an air inlet, an air outlet, and a main filter.

According to this invention, the main filter included in the housing of the air purifying device acts as an adsorption and an absorption filter, and it comprises at least a mixture of particles of activated carbon (AC) and a plurality of filament-like support elements, preferably fibres, and an adhesive or bonding agent at least partially coating the filament-like elements, wherein the particles of AC have an average particle size between 0.4 and 200 microns, preferably between 0.4 and 150 microns.

Due to the size of the particles of AC used, they adhere to the plurality of fibres in a sponge-like mesh configuration, the particles of carbon remaining attached to the plurality of fibres by at least coating the fibres with the adhesive or bonding agent and in some embodiments by the appearance of an electrostatic force interaction between the particles and the fibres to which they are adhered, allowing the particles of AC to fully impregnate, mix and intersperse with the fibres used.

Moreover, as mentioned previously, the air impeller is controlled to ensure an air speed of up to a given speed through the sponge-like mesh, ensuring that the particles remain attached to the filament-like elements.

In a preferred embodiment of the present invention, the main filter further comprises a limiting layer covering or over at least one of:
- a first side of the main filter facing the air inlet of the air purifying device, or
- a second side of the main filter facing the air outlet of the air purifying device.

The limiting layer provides the main filter with pre-emptive safety measures, which will retain the components that form the main filter, in the event of the main filter sustaining damages or certain particles being released, and therefore making sure they don't enter the airflow going through the air purifying device.

Optionally, in certain embodiments of the air purifying device, it further comprises at least one of: an HEPA filter, a mechanical particle retention pre-filter, or one or more secondary filters, wherein one or more of the secondary filters are for specific pollutants such as VOCs (including aldehydes, amines, carboxylic acids and thiols).

In preferred embodiments, the sponge-like mesh of the main filter may also include a reducing agent (specifically a reducing metal), in addition to the particles of AC, which may be used in the sponge-like mesh either as:
a) a powdered metal with an average particle size between 0.4 microns and 200 microns, which is adhered to the plurality of fibres, or
b) metal wires, foils, shavings or granules, depending on the commercially available forms of each reducing metal.

In those embodiments where a reactive metal is added to the sponge-like mesh of the main filter, it is advisable to prepare the sponge-like mesh by addition of the less active metals as a fine powder, whereas the more active metals (pyrophoric) should be added to the filter in the form of wires or granules.

The partial use of the cited adhesive agent on the fibres, in order to give consistency to the filaments (increasing the mesh feature) and to increase the retention of particles of AC and the reducing agent, will allow to attain higher flows of the contaminated air, when necessary. The adhesive is used in minimum quantities so that the adhesive coating layer on the fibres has a minimum effect on the effective working surface of the dispersed particles of AC and the reducing agent on the fibres of the sponge-like mesh. Therefore, the preferred percentage in weight of adhesive in the sponge-like mesh is between 1% and 7% of the total weight of the other materials which form the sponge-like mesh. As such, the percentage in weight of the fibres, particles of AC and in certain preferred embodiments particles of the reducing agent is between 93% and 99% of the total weight of the sponge-like mesh configuration.

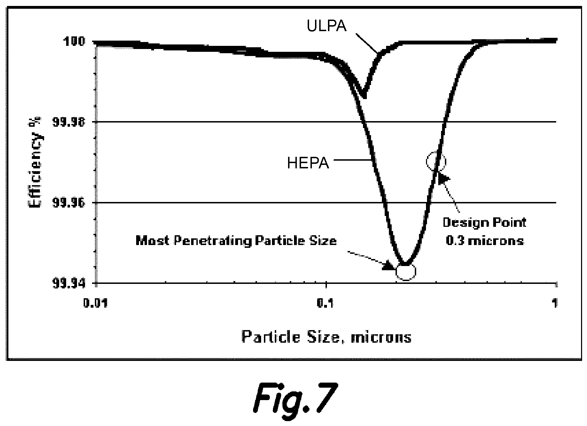

The average size of the particles of AC and the reducing metal in the sponge-like mesh configuration, are set at a minimum size of 0.40 microns. This minimum size has been chosen due to the decrease in efficiency that appears in High Efficiency Particulate Air (HEPA) filters, but also in Ultra-Low Particulate Air (ULPA) filters, as seen in the graph in FIG. 7. In this figure, the plotted lines in the graph show that the efficiency of HEPA and ULPA filters decreases slightly when the size of the particles to be retained or captured (filtered) are between 0.10 and 0.40 microns, specifically between 0.16 and 0.30 microns for HEPA filters, and approximately 0.12 microns in the case of ULPA filters. For this reason values under 0.40 microns have been disregarded.

Adsorption and absorption are both sorption processes, which involve the occurrences of a physical and/or chemical process or reaction in the main filter, wherein:
- adsorption is defined as the adhesion of atoms, molecules or particles of a fluid or dissolved solid (also known as adsorbate) to a surface, specifically the surface of AC particles due to its porosity and high internal area (also known as adsorbent), whereas
- absorption is defined as a physicochemical process or mainly a chemical process in which an atoms or molecules (as gases or vapours) are retained throughout the volume of an absorbent (in this particular case, the sponge-like mesh containing particles of AC and the reducing metal), specifically through chemical absorptions or reactions, which in some cases may be combined with physical interactions (nonreactive).

In a preferred embodiment of the invention, the air impeller is controlled to ensure an air speed of up to 10 m/s through the sponge-like mesh, as well as the rest of the filters in the air purifying device, retaining the fibres or filament-like elements, the particles of AC and the reducing metal of the sponge-like mesh, by means of the adhesive agent used.

This speed limit guaranties that the particles of both AC and the reducing agent remain attached or adhered to the plurality of fibres, by the combined efforts of the adhesive agent coating the fibres (as well as the particles in some cases) and the electrostatic interaction with the fibres.

The adhesive used to coat the filament-like elements in the sponge-like mesh configuration is selected from a group comprising: protein-based glues, polyurethanes, polyolefins, polyamides (nylon-like glues), polyesters, silicones, cellulose ethyl ethers (such as ethyl cellulose, but not cellulose itself), carboxymethylcellulose and polyvinylpyrrolidine. In other words, examples of almost all classes of commercially available adhesives or glues are useful, with a few practical exceptions, which are those that may react with some contaminants to potentially afford toxic or hazardous derivatives.

Thus, the use of certain polymers, such as the following glue types, are contraindicated: (a) those containing many hydroxy groups, such as cellulose (untreated cotton) and several subclasses of epoxy resins (once polymerized or copolymerized), which may be converted into polynitrates; (b) those containing double bonds (e.g., arising from rubber), which may be cleaved by ozone; (c) those based on polymethacrylates and polyacrylonitriles, which under certain conditions might decompose releasing traces of methanol or hydrogen cyanide, respectively; and (d) specially those polymers that are made from formaldehyde, such as phenol-formaldehyde, urea-formaldehyde and melamine-formaldehyde resins, which can release trace amounts of formaldehyde, a very toxic VOC, if they have not been prepared under optimal conditions and/or by decomposition mediated by contaminants of the airflow.

Preferably, the reducing agent is an active reducing metal with a standard electrode potential between -2.4 V and -0.4 V. Metals with a standard electrode potential higher than magnesium (Mg; -2.372 V), are not advisable due to their high reactivity, and metals with a standard electrode potential lower than iron (Fe; -0.44 V) are not useful as they are not capable of capturing acids and inorganic anhydrides. Therefore, the active reducing metal is preferably selected from a group comprising (in ascending order of standard electrode potential): magnesium (Mg), aluminium (Al), zirconium (Zr), titanium (Ti), manganese (Mn), vanadium (V), zinc (Zn), chromium (Cr) and iron (Fe) as well as alloys or mixtures thereof, wherein the preferred reducing metal is zinc (Zn).

With regards to the plurality of fibres used to obtain the sponge-like mesh of the main filter, these fibres may be inorganic fibres, synthetic fibres, natural fibres or mixtures thereof, and they should preferably be neutral and non-hygroscopic.

In the case of inorganic fibres, these are usually selected from a group including glass fibres (also known as glass wool), mineral wool fibres (also known as rock or stone wool, which are silicates), ceramic wool fibres (which are a type of synthetic mineral wool fibres, also known as kaowool), and mixtures thereof. Preferably, the inorganic fibres used are either glass wool or rock wool.

With regards to the term silicates when describing mineral wool fibres, it should be pointed out that only those natural silicates that are not bio-persistent and that have been proved as not causing pulmonary lesions must be employed. Therefore certain inorganic materials, which are too fibrous and silky, specifically Asbestos fibres (amianthus), are highly unadvisable due to their carcinogenic behaviour, as well as natural zeolite fibres, which are known to cause pulmonary lesions. Despite the fact that certain embodiments of the air purifying device may include an HEPA filter which would stop small fibrous and flaky particles that could be generated from these hazardous materials, it is still highly recommended to avoid the use of these materials.

In the case of synthetic fibres, these are usually selected from a group including: (a) polyesters, such as polyethylene terephthalate (PET) or similar copolymers such as polybutylene terephthalate (PBT) or polyethyl acrylate (PEA), (b) polyamides (PA) such as nylon-6, nylon-66, etc., as well as amides of terephthalic acid, (c) polypropylene (PP) and other similar alkene-derived polymers or copolymers, and mixtures thereof. The preferred synthetic fibre is a polyamide fibre, specifically nylons.

However, the use of certain synthetic fibres, such as those made from acrylonitrile or styrene, is not advisable due to their composition and the plausible reaction of these synthetic fibres with some of the contaminating gases or vapours captured by the filter, with the possibility of generating small amounts of hazardous or toxic by-products.

Furthermore, the use of filamentous polymers made from formaldehyde (methanal), such as phenol-formaldehyde resins or urea-formaldehyde resins, should also be avoided as they are capable of releasing traces of toxic formaldehyde by reacting with some of the contaminants, and the use of this kind of synthetic fibres would require the use of an additional filter to capture said toxic formaldehyde.

Finally, in the case of natural fibres (of animal or plant origin), these are usually selected from a group including wool, silk, hair, collagen, keratin, animal proteins and mixtures or simple derivatives thereof. The use of semisynthetic fibres comprising chemically modified natural fibres may also be used. The preferred natural fibre is wool, specifically undyed sheep wool.

However, the use of certain natural fibres should be avoided. Among these fibres we can mention natural cellulose fibres (cotton) and its semisynthetic derivatives (cellulose acetate, viscose), or chitin and chitosan and their derivatives, to prevent both the appearance of mould inside the main filter and the formation of undesired by-products (polynitrates) by reaction with the trapped contaminants, specifically NO₂.

Regardless of the type of fibre used in the main filter to obtain the sponge-like mesh, the plurality of fibres are selected from amongst short fibres, long fibres or a mixture thereof, with a fibre length between 1 mm and 15 cm and a diameter of the fibres between 0.4 and 200 microns.

It should be noted that, when an adhesive agent is used to at least partially coat the plurality of fibres of the sponge-like mesh to retain particles of AC, and in certain embodiments, particles of the reducing agent, the particle size isn't relevant as long as the adhesive agent is strong enough to retain these particles. However, in a preferred embodiment, the particle size of the particles AC and the reducing agent added to the fibres of the main filter are smaller or equal to said diameter of the fibres, favouring the appearance of a strong electrostatic force interaction between the fibres and the particles used in the sponge-like mesh configuration.

In a preferred embodiment of the main filter, the sponge-like mesh configuration uses between 1% and 7% of the total weight of the adhesive agent, and between 93% and 99% of fibres and particles of AC and the reducing agent, in relatively or about equal proportions, from the total weight of the sponge-like mesh. In the absence of the particles of the reducing agent, in certain embodiments, the proportion of particles of AC may increase proportionately. However, this mixture may vary according to the following w/w ratio: 1.0-4.0 of filament-like elements : 0.5-2.0 of particles of AC : 0.2-1.0 of the reducing metal, to obtain the desired sponge-like mesh.

For example, a ratio of 2.0 g of fibres to 1.0 g of AC particles is optimal, and between 0.2 g and 0.5 g of the reducing metal is added, depending on its particle size and its activity. In the case of zinc, 0.5 g is used.

In a preferred embodiment of the present invention, the sponge-like mesh of the main filter comprises particles of AC of approximately 100 microns, particles of zinc as the reducing metal of approximately 150 microns, and either glass wool, nylon filaments or undyed sheep wool. Due to the porosity of the particles of AC, they are capable of adsorbing gas molecules of O₃ and NO₂, which react immediately and irreversibly with functional groups and substituents of AC, wherein:
- ozone (O₃), gives ozonides and other oxidation groups, and
- nitrogen dioxide (NO₂, which is in equilibrium with its dimer N₂O₄, inside the pores of AC particles) nitrates irreversibly with the most activated aromatic rings of AC.

However, nitric oxide (NO) and sulphur oxides, specifically sulphur dioxide (SO₂) don't react with particles of AC, and they are only partially and reversibly adsorbed, but in the long range they are displaced by other gases (N₂ or O₂).

That is why the use of a reducing metal in the sponge-like mesh is crucial for trapping or capturing certain contaminating gases or vapours, wherein the preferred reducing metal, as stated previously, is zinc (Zn) which is capable of capturing any volatile acid or inorganic anhydride which can produce an acid when it contacts the humidity of air. Therefore, zinc particles yield zinc salts and hydrogen gas when they react, and consequently capturing:
- nitrous (HNO₂) or nitric (HNO₃) acid due to the presence of N₂O₃ (NO + NO₂), N₂O₄ (dimer of NO₂), and/or any trace of N₂O₅, provided that there is water vapour in the airflow (humid air),
- sulphur dioxide (SO₂) in its hydrated form sulphurous acid (H₂SO₃), and its respective oxidation product sulphur trioxide (SO₃; sulphuric anhydride) which appears due to the quick reaction of SO₂ with O₃ and NO₂ or by reacting slowly with atmospheric O₂. When SO₃ reacts spontaneously with humid air, it turns into sulphuric acid (H₂SO₄) one of the main constituents of acid rain, and
- Volatile Organic Compounds (VOCs) with acidic properties.

Furthermore, as indicated previously, when O₃ and NO₂ react with AC, oxidation and nitration by-products are formed. The mixture of particles of the reducing metal and particles of AC, can avoid the accumulation of these by-products on the superficial areas of AC.

In general, the main filter is non-reusable and once it has reached the end of its life expectancy it must be disposed of appropriately, preferably through incineration.

The air purifying device further comprises a telematics system connected to an external control unit, in order to access live data regarding contamination levels of a specific geolocation common to the air purifying device. A processor unit included in the telematics system determines if the air purifying device has to be activated according to the contamination levels of said geolocation. The air impeller is also controlled by the cited processor unit, according to a plurality of working regimes.

It will also be understood that any range of values given may not be optimal in extreme values or under extreme conditions, and may require adaptations of the invention when these extreme values are applicable, such adaptations being within reach of a skilled person.

Other features of the invention appear from the following detailed description of an embodiment.

### Brief description of the Figures

The foregoing and other advantages and features will be more fully understood from the following detailed description of an embodiment with reference to the accompanying drawings, to be taken in an illustrative and non-limitative manner, in which:
- **FIG. 1** is a schematic representation of a preferred first embodiment of the air purifying device;
- **FIG. 2** is a schematic representation of a preferred second embodiment of the air purifying device;
- Both **FIG. 3a** and **FIG. 3b** illustrate a preferred embodiment of an interior structure of the air purifying device;
- **FIG. 4** is a schematic representation of the telematics system, which connects the air purifying device to an external control unit;
- **FIG. 5** is a simplified close-up view of the sponge-like mesh, which composes the main filter;
- **FIG. 6** is a close-up view of a section of FIG. 5 which illustrates an adhesive coating the fibres of the sponge-like mesh configuration; and
- **FIG. 7** is a graph representing the efficiency of both known HEPA and ULPA filters for particle sizes between 0.01 microns and 1 micron.

### Detailed description of an embodiment

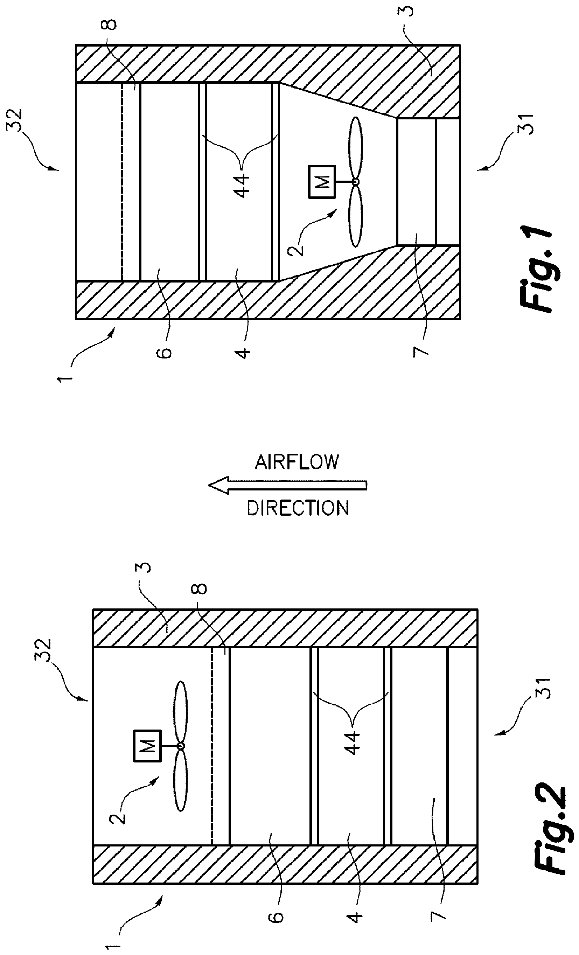

The foregoing and other advantages and features will be more fully understood from the following detailed description of an embodiment with reference to the accompanying drawings, to be taken in an illustrative and not limitative, in which:
In the schematic representation of an air purifying device, 1, in both **FIG. 1** and **FIG. 2****,** both air purifying devices comprise at least: a housing 3, which in itself comprises an air impeller device 2, an air inlet 31, an air outlet 32 and a main filter 4.

However, in a first preferred embodiment of the air purifying device 1, as seen in **FIG. 1****,** the air purifying device is arranged such that the airflow going through the housing 3 of the air purifier 1 goes through a plurality of filters arranged in a set configuration in order to ensure the maximum effect whilst filtering and cleansing the airflow.

As soon as the airflow enters the housing 3 of the purifying device 1, through the air inlet 31, the air flows through a mechanical pre-filter 7, whose primary role is to filter and retain large airborne particles, for example dust, pollen or heavy metals. Mechanical pre-filters 7, are usually coarse or low pressure filters, such as those according to standard EN779:2012 which are classified according to their level of filtration from G1 to G4, and are suitable for filtering airborne particles ≥ 10 microns (µm) such as leaves, insects, sand, ashes and pollen for example.

The air impeller device, 2, usually an aspirator device or a centrifugal fan operated by a motor (or an equivalent actuator), M, is placed straight after the mechanical pre-filter 7 and directs the airflow towards another set of filters, specifically the main filter 4 and an HEPA filter 6, in this order before leaving the housing 3 through air outlet 32.

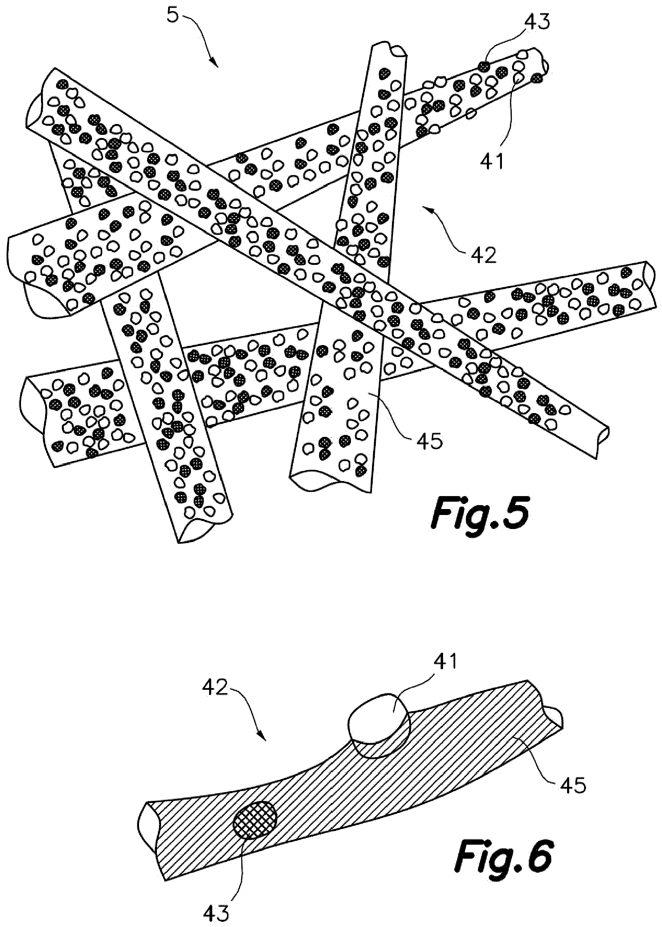

The main filter 4 comprises a sponge-like mesh 5 made up of particles of AC 41 adhered to a plurality of filament-like elements 42, and in some embodiments it includes particles of a reducing metal 43, all three materials preferably in equal proportions, as well as an adhesive agent 45 coating the filament-like elements 42. However, in alternative embodiments, these three materials may be according to the following w/w ratio 2.0 : 1.0 : 0.5 (fibres 42 / particles of AC 41 / the reducing metal 43, preferably Zn). Other ratios may be useful depending on the main contaminants of the airflow, the reactiveness of the metal used as the reducing metal 43, e.g. between 1.0-4.0 : 0.5-2.0 : 0.2-1.0 (fibres 42 / particles of AC 41 / the reducing metal 43), wherein the filters will be adapted to the needs of the user (from different areas, towns, industrial surroundings, climates, etc.). This sponge-like mesh 5 will be further discussed, in detail, with reference to **FIG. 5****.** The purpose of this main filter 4 is to reduce the concentration of gases which are harmful to humans, mainly ozone (O₃), and nitrogen dioxide (NO₂), and in some cases sulphur oxides (SOₓ). The use of particles of AC 41 in this main filter 4 can also partially or temporarily adsorb bad odours, as well as destroy acidic VOCs when it includes a reducing metal 43. In some embodiments, the main filter 4 further comprises a limiting layer 44 on either one or both sides of the main filter 4 facing the air inlet 31 or the air outlet 32.

Once the flow of air has gone through the main filter 4, it is then filtered by the HEPA filter 6 placed before the air outlet 32 of the housing 3 of the purifying device 1. The HEPA filter 6, as is known in the state of the art, is made up of a mat of fibres, preferably glass wool fibres, and is tasked with removing any remaining particles from the airflow which are above and below particles of a size of 0.3 ± 0.1 microns in size, wherein the particles stick to the fibres, through interception, impaction or diffusion.

It should be noted that HEPA filters 6 are unable to filter or remove gas concentrations or odour molecules (for instance, VOCs which cause bad smells) from a stream or flow of air, but they are excellent when it comes to trapping or capturing coarse airborne particles (i.e. PM_{2.5} and PM₁₀) and that additional secondary filters 8, may be added to the air purifying device 1 in order to achieve this, wherein the secondary filters 8 used should contain specific absorbents for toxic inorganic gases and for VOCs (both toxic or those that cause bad odours).

Whereas, in a second preferred embodiment of the air purifying device 1, as seen in **FIG. 2****,** the air impeller device 2, is placed just before the air outlet 32 of the housing 3 of the air purifying device 1, and the other remaining filters are placed consecutively after the air inlet 31, wherein the first filter is the mechanical pre-filter 7, the second filter is the main filter 4, and the last filter placed before the air impeller 2 is the HEPA filter 6.

As in the embodiment described in **FIG. 1****,** this particular embodiment can also include additional secondary filters 8, containing appropriate components for trapping molecules that are toxic and/or cause bad odours, either inorganic or VOCs.

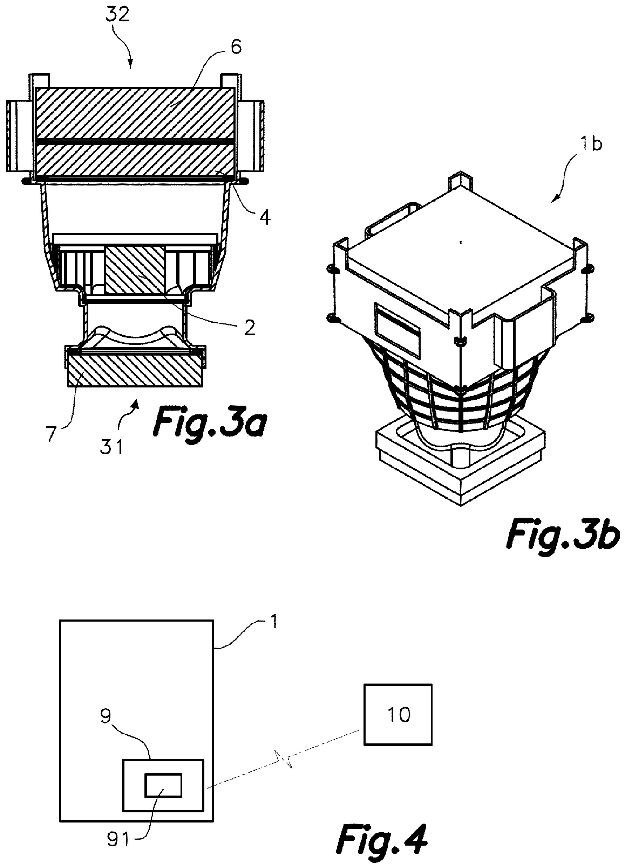

Both **FIG. 3a** and **FIG. 3b****,** illustrate a preferred embodiment of an interior structure 1b of the air purifying device 1, which doesn't include the housing 3, wherein **FIG. 3a** is a front view of an interior central portion of the interior structure 1b, which shows the location of the interior supports for the different filters, the air inlet 31 and outlet 32, and the location of the air impeller device 2. The different components of the air purifying device 1 are arranged in the same manner as those in **FIG. 1****.** The mechanical pre-filter 7 is located straight after the air inlet 31 and the following elements are arranged consecutively, in the following order: the air impeller device 2, the main filter 4 and the HEPA filter 6, arranged before the air outlet 32. **FIG. 3b****,** only illustrates a preferred embodiment of the exterior shape of the interior structure 1b of the air purifying device 1.

**FIG. 4** illustrates a schematic representation of a telematics system 9 which may be installed in certain preferred embodiments of an air purifying device 1 according to the invention which connects the air purifying device 1 to an external control unit 10, enabling a bidirectional wireless communication between them. The telematics system 9 receives record information regarding contamination levels from the external control unit 10, wherein the record information is preferably live data of a specific geolocation in which the air purifying device 1 is installed.

In a preferred embodiment, the telematics system 9 installed in the air purifying device 1 is a control panel which includes at least:
- a processor 91, which processes the live data regarding contamination levels sent from the external control unit 10;
- a memory 92, which stores a plurality of predetermined executable working regimes for the air purifying device, wherein an optimum working regime is selected from the plurality of predetermined executable working regimes according to the live data regarding contamination levels analysed by said processor 91; and
- a display 93, which allows a user to view, modify or select data regarding the air purifying device 1 such as for example: if it is operational (if it has to be activated or not), the working regime currently in use, allow the user to select any of the predetermined working regimes or alert when the filters have to be swapped through an audio-visual message, among others.

The motor M of the air impeller 2 of the air purifying device 1 is controlled, by the processor 91, according to the working regime currently in use at any time which has been selected by the telematics system 9, according to the live data regarding contamination levels in the environment, or the user through the display 93, which in turn regulates the airflow going through the air purifying device 1.

As stated previously, **FIG. 5** illustrates a close-up or detailed view of the sponge-like mesh 5 which composes the main filter 4 of the air purifying device 1.

In one embodiment of the air purifying device 1, the sponge-like mesh 5 of the main filter 4 is made up of particles of AC 41, a plurality of fibres 42 (which are preferably either glass wool, rock wool, nylons or undyed wool yarn), and an adhesive agent 45 coating the fibres 42 (which has been referenced in the illustration but it hasn't been identified visibly, unlike **FIG. 6**), wherein the particles of AC 41 have a size between 0.4 and 200 microns and the fibres 42 are preferably glass or rock wool, nylon-6,6 or undyed sheep wool. The sponge-like mesh 5 is obtained by dispersing the particles of AC 41 through the fibres 42, wherein said particles 41 adhere to the fibres 42 mainly by adhering to the coating layer of the adhesive 45 on the fibres 42, and in certain embodiments by an electrostatic force interaction between the particles of AC 41 and the fibres 42.

In preferred embodiments of the air purifying device 1, the sponge-like mesh 5 of the main filter 4 further comprises particles of a reducing metal 43, wherein the particles of the reducing metal 43 have a size between 0.4 and 200 microns, and the reducing metal is preferably an active reducing metal, preferably zinc.

Furthermore, the use of the particles of AC 41, particles of the reducing metal 43 and a plurality of fibres 42 are used in equal proportions without taking into account the proportion of adhesive 45 used to coat the fibres 42, in the sponge-like mesh 5 configuration. In embodiments in which the reducing metal 43 isn't used in the sponge-like mesh 5, the proportion of particles of AC 41 may increase proportionately.

It should be noted that the flow of air to be filtered, conveyed or directed by the air impeller device 2 is controlled to provide a limited maximum speed provided by the motor M, so that the particles of AC 41 or the reducing metal 43 remain adhered or attached to the plurality of fibres 42 of the sponge-like mesh 5, when the airflow goes through the main filter 4.

The size and distribution of the particles of AC 41 and the reducing metal 43 adhered to the fibres 42 in **FIG. 5** have been exaggerated and amplified in order to simplify and better understand the composition of the sponge-like mesh 5 of the main filter 4. However it must be noted, that the actual sponge-like mesh 5 comprises a homogenous mixture, preferably in equal proportions or in a 2.0:1.0:0.5 w/w ratio, of fibres 42 and particles of AC 41 and the reducing metal 43, wherein the particles 41, 43 adhere to and cover the whole surface of the fibres 42 coated with the adhesive agent 45 (which hasn't been represented to simplify the illustration, unlike **FIG. 6** where the fibre 42 has been covered with a pattern of slanted parallel lines to represent the adhesive 45).

Finally, **FIG. 6** is a close-up view of a random section of the sponge-like mesh 5 illustrated in **FIG. 5****,** which illustrates an adhesive 45 coating a fibre 42 of the sponge-like mesh 5 configuration, wherein the particles of activated carbon 41 and the reducing metal 43, are only partially covered, leaving the maximum effective working surface available for the main filter 4 to operate under optimum conditions.

It will be understood that various parts of one embodiment of the invention can be freely combined with parts described in other embodiments, even being said combination not explicitly described, provided there is no harm in such combination.

In particular the so called filament-like support elements used in this patent application could embrace a very broad range of elements other than fibres and the use of which will appear obvious in light of the teaching of this invention.

## Claims

1. An air purifying device (1), through which a controlled flow of air is conveyed to be filtered, said air purifying device (1) including an air impeller device (2) providing said controlled flow of air and having a housing (3) which comprises:
- an air inlet (31) and an air outlet (32);
- a main filter (4) comprising at least a mixture of:
∘ particles of activated carbon (41),
∘ a plurality of filament-like elements (42), and
∘ an adhesive agent (45);
**characterized in that:**
- said particles of activated carbon (41) have an average particle size between 0.4 and 200 microns,
- said filament-like elements (42) are of an organic or inorganic nature, either natural or synthetic, and
- the particles of activated carbon (41) are adhered to said plurality of filament-like elements (42) in a sponge-like mesh configuration (5) by at least partially coating the filament-like elements (42) with said adhesive agent (45),
wherein the percentage in weight of adhesive (45) in the sponge-like mesh (5) is between 1% and 7% out of the total weight of the sponge-like mesh (5),
wherein said air impeller (2) is controlled to provide an air to flow through the sponge-like mesh (5) at a speed of up to 10 m/s without displacing the particles of activated carbon (41) or the filament-like elements (42), and
wherein said main filter (4) acts as an adsorption and an absorption filter.

2. The air purifying device (1) according to claim 1, wherein the adhesive (45) used to coat the filament-like elements (42) is selected from a group comprising: protein-based glues, polyurethanes, polyolefins, polyamides including nylon-like glues, polyesters, silicones, cellulose ethyl ethers, carboxymethylcellulose and polyvinylpyrrolidine.

3. The air purifying device (1) according to claim 1, wherein the main filter (4) further comprises a reducing metal (43) which is adhered to the plurality of filament-like elements (42), wherein said reducing metal is in the form of particles with an average size between 0.4 and 200 microns.

4. The air purifying device (1) according to claim 3, wherein the reducing metal (43) is an active reducing metal with a standard electrode potential between -2.4 V and -0.4 V, preferably selected from a group comprising: magnesium, aluminium, zirconium, titanium, manganese, vanadium, zinc, chromium and iron, as well as alloys or mixtures thereof.

5. The air purifying device (1) according to claim 1, wherein the filament-like elements (42) of the main filter (4) are inorganic fibres selected from a group comprising: glass wool, mineral wool, ceramic wool, and mixtures thereof.

6. The air purifying device (1) according to claims 1 or 5, wherein the filament-like elements (42) of the main filter (4) are synthetic fibres selected from a group comprising: polyesters, polyamides, polypropylenes, similar copolymers and mixtures thereof.

7. The air purifying device (1) according to claims 1, 5 or 6, wherein the filament-like elements (42) of the main filter (4) are natural, animal-based or plant-based, fibres selected from a group comprising: wool, silk, hair, collagen, keratin, or other animal proteins and mixtures thereof.

8. The air purifying device (1) according to claim 5, 6 or 7, wherein the plurality of filament-like elements (42) are selected from among short fibres, long fibres or a mixture thereof, with a fibre length between 1 mm and 15 cm and a diameter between 0.4 and 200 microns.

9. The air purifying device (1) according to claim 1, 3 or 8, wherein the particle size of the activated carbon (41) or the reducing metal (43) added to the fibres of the main filter (4) are smaller or equal to said diameter of the fibres.

10. The air purifying device (1) according to claim 1, wherein the main filter (4) further comprises a limiting layer (44) on at least one of:
- a first side of the main filter (4) facing the air inlet (31) of the air purifying device (1), or
- a second side of the main filter (4) facing the air outlet (32) of the air purifying device (1).

11. The air purifying device (1) according to claim 1 or 10, wherein the air purifying device (1) further comprises at least one of:
- an HEPA filter (6), before the air outlet (32),
- a mechanical particle retention pre-filter (7), after the air inlet (31), or
- one or more secondary filters (8) after the main filter (4),
wherein said one or more secondary filters (8) are for specific pollutants including toxic VOCs or bad odours caused by VOCs.

12. The air purifying device (1) according to any of the previous claims, wherein the particles of activated carbon (41), the filament-like elements (42) and the particles of the reducing metal (43) included in the main filter (4) are used, respectively, in either:
- relatively equal proportions, or
- a w/w ratio of 1.0 : 2.0 : 0.5.

13. The air purifying device (1) according to any of the previous claims, wherein the air purifying device (1) further comprises a telematics system (9) connected to an external control unit (10), in order to access live data regarding contamination levels of a specific geolocation common to the air purifying device (1).

14. The air purifying device (1) according claim 13, wherein a processor unit (91) included in the telematics system (9) determines if the air purifying device (1) has to be activated according to the contamination levels of said geolocation.

15. The air purifying device (1) according to claim 14, wherein the air impeller (2) is also controlled by said processor unit (91), according to a plurality of working regimes.

16. The air purifying device (1) according to any of the previous claims, wherein the main filter (4) further comprises a reducing metal (43) which is adhered to the plurality of filament-like elements (42), wherein said reducing metal (43) is in the form of wires, foils, shavings or granules.
